# EUROPEAN PATENT SPECIFICATION

(11) **EP 3 606 936 B1**
(45) Date of publication and mention of the grant of the patent: **20.10.2021**
(21) Application number: 18715649.2
(22) Date of filing: 03.04.2018
(51) Int. Cl.: C07K 1/14, C12N 9/26, C12N 9/54

(54) **RECOVERY PROCESS**
VERFAHREN ZUR RÜCKGEWINNUNG
PROCÉDÉ DE RÉCUPÉRATION

(30) Priority: 03.04.2017 EP 17164452
(43) Date of publication of application: 12.02.2020
(73) Proprietor: Novozymes A/S, 2880 Bagsvaerd (DK)
(72) Inventor: GLANVILLE, Simon, 2880 Bagsvaerd (DK); PIND, Peter, Frode, 2880 Bagsvaerd (DK); JAKOBSEN, Sune, 2880 Bagsvaerd (DK); JOHANSEN, Lars, 2880 Bagsvaerd (DK); JACOBSEN, Carsten, 2880 Bagsvaerd (DK); ANDERSEN, Kim, Bruno, 2880 Bagsvaerd (DK); BEIER, Søren, Prip, 2880 Bagsvaerd (DK); RYPE, Jens, Ulrik, 2880 Bagsvaerd (DK)
(74) Representative: NZ EPO Representatives
(86) International application number: PCT/EP2018/058387
(87) International publication number: WO 2018/185048

(56) References cited:
- EP-A1- 2 125 865
- EP-A2- 1 456 361
- WO-A1-92/13954
- WO-A1-02/055539
- WO-A2-00/11890
- US-B1- 6 316 240
- PALMER IRA ET AL: "Preparation and extraction of insoluble (Inclusion-body) proteins from Escherichia coli", CURRENT PROTOCOLS IN PROTEIN SCIENCE, JOHN WILEY & SONS, INC, US, vol. 1, no. SUPPL.70, 1 November 2012 (2012-11-01), pages 6.3.1-6.3.20, XP002731972, ISSN: 1934-3655, DOI: 10.1002/0471140864

## Description

### Reference to sequence listing

This application contains a Sequence Listing in computer readable form. The computer readable form is incorporated herein by reference.

### FIELD OF THE INVENTION

The present invention relates to an improved method for solubilizing protein crystals and/or protein precipitate in a fermentation broth.

### BACKGROUND OF THE INVENTION

The fermentation yield of industrial proteins, e.g. enzymes such as proteases, has increased dramatically in the recent years. In many industrial processes, the concentration of the protein in the fermentation broth is so high that a significant part of the protein is found in form of crystals or solid precipitates at the end of the fermentation process.

WO93/13125 discloses a process for producing protease where the protease is precipitated during fermentation by adding a precipitating agent to the production medium. It was found that precipitating protease during fermentation protects the protease against proteolysis.

For many fermentation products it is desired that the microorganism used in fermentation is removed from the product. One of the first steps in the product recovery process is usually removal of the cellular biomass from the product, for example by filtration or centrifugation removing the solid cellular biomass from the liquid comprising the desired product. This step is often called the primary separation step where the microorganism is separated from the liquid fraction, and the liquid fraction can subsequently be processed with one or more downstream steps, such as ultrafiltration, stabilisation, spray drying, granulation which eventually will turn the desired product into the form and concentration that is intended for this particular product.

However, if part of the desired product is present in solid form after the fermentation, suitable measures must be taken to solubilize the product before the biomass is removed from the liquid fraction comprising the product.

US 6,316,240 discloses a process for solubilizing enzymes precipitated during fermentation process where the pH of the culture broth is adjusted to a pH between 9.5 and 13.0. The process is exemplified with an amylase fermentation.

EP 2 125 865 discloses a process for solubilizing protease crystals and/or protease precipitate in a fermentation broth comprising diluting the fermentation broth 100-2000 % (w/w); adding a divalent salt; and adjusting the pH value of the fermentation broth to a pH value below 5.5.

EP 1 456 361 discloses a process for harvesting crystalline particles, in particular crystalline alpha-amylase or protease, from fermentation broth, wherein the fermentation broth is separated into a biomass fraction, a crystalline alpha-amylase or protease fraction and a supernatant fraction.

### SUMMARY OF THE INVENTION

The invention provides a method for recovering a desired product from a fermentation broth, where the desired product is present in precipitated form in the fermentation broth, the method comprising:
a) a first separation step separating the fermentation broth in a first phase and a second phase, wherein the first phase comprises supernatant from the fermentation broth and it may comprise a part of the cells and cell debris from the fermentation broth, and the second phase comprises the desired product in precipitated form, cells and cell debris; and
b) a solubilisation step where the desired product in precipitated form is solubilized, wherein the one or more enzyme is selected among proteases having at least 80% sequence identity, such as at least 85% sequence identity, such as at least 95% sequence identity, such as at least 96% sequence identity, such as at least 97% sequence identity, such as at least 98% sequence identity, such as at least 99% sequence identity to one of SEQ ID NO: 1- 6, or selected among amylases having at least 80% sequence identity, such as at least 85% sequence identity, such as at least 95% sequence identity, such as at least 96% sequence identity, such as at least 97% sequence identity, such as at least 98% sequence identity, such as at least 99% sequence identity to one of SEQ ID NO: 7-9.

In one preferred embodiment the method further comprise a second separation step where the solubilized desired product from step b) is separated from the cells and/or cell debris.

### DETAILED DESCRIPTION OF THE SEQUENCES

SEQ ID NO: 1 : amino acid sequence of *B*. *lentus* protease (Savinase)
SEQ ID NO: 2 : amino acid sequence of *B*. *amyloliquefaciens* protease (BPN')
SEQ ID NO: 3: Subtilisin Carlsberg
SEQ ID NO: 4: Protease from *Bacillus sp.* TY145, disclosed in WO 92/17577
SEQ ID NO: 5: Protease from *Nocardiopsis* sp. NRRL 18262, disclosed in WO 88/03947
SEQ ID NO: 6: *Pyrococcus furiosus* protease, disclosed in US 6,358,726.
SEQ ID NO: 7: Alpha-amylase from *Bacillus sp.* disclosed in WO 2000/060060.
SEQ ID NO: 8: Alpha-amylase from *Bacillus stearothermophilus* SEQ ID NO: 9: Alpha-amylase from *Bacillus licheniformis*

### SHORT DESCRIPTION OF THE DRAWINGS

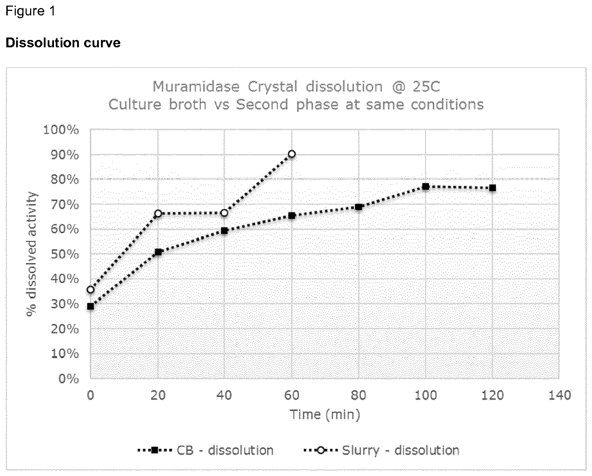

Figure 1 shows the dissolution of muramidase from culture broth (CB) or from the second phase according to the invention, reflecting the results of the experiments in example 5.

### DETAILED DESCRIPTION OF THE INVENTION

The present invention relates to the primary separation of a fermentation broth where part of the desired fermentation product is present in solid form such as in crystalline or amorphous form. It is not important for the present invention whether the desired fermentation product is in crystalline or amorphous form or even in a mixture of these forms, what is important is that a significant part of the desired product is in solid form and therefore can the primary separation step not readily be performed as a simple solid/liquid separation process. The primary separation step has the purpose of removing the cells and cell debris from the desired product.

The invention relates to a method of recovering a desired fermentation product from a fermentation broth, wherein the fermentation product is present in at least partially in insoluble form in the fermentation broth such as in crystalline or amorphous form; comprising:
- a first separation step separating the fermentation broth in a first phase and a second phase, wherein the first phase comprising liquid from the fermentation broth and desired fermentation product in soluble form; and the second phase comprises the desired fermentation product in insoluble form, cells and/or cell debris; and
- a solubilisation step, wherein the desired fermentation product in the second phase is solubilized, wherein the one or more enzyme is selected among proteases having at least 80% sequence identity, such as at least 85% sequence identity, such as at least 95% sequence identity, such as at least 96% sequence identity, such as at least 97% sequence identity, such as at least 98% sequence identity, such as at least 99% sequence identity to one of SEQ ID NO: 1 - 6, or selected among amylases having at least 80% sequence identity, such as at least 85% sequence identity, such as at least 95% sequence identity, such as at least 96% sequence identity, such as at least 97% sequence identity, such as at least 98% sequence identity, such as at least 99% sequence identity to one of SEQ ID NO: 7-9.

In some embodiments the invention relates to a method further comprising a second separation step separating the liquid phase comprising of the solubilized desired fermentation product from the solid biomass, optionally after mixing the solubilized first phase with part or all of the first phase from the first separation step.

The invention is based on the observation that fermentation broth appears to contain some components that are detrimental to the solubilisation process. Data seems to indicate that optimal conditions for high solubility for proteins such as enzymes, are conditions with high enzyme concentration and low ion concentration. However, it should be understood that the invention is not limited by any particular theory. It has been found beneficial if the solubilisation of the desired product can take place in the absence of at least part of the liquid part of the fermentation broth containing the majority of these disadvantageous components.

According to the invention fermentation is intended to mean the process where one or more microorganisms are grown in a fermenter in a fermentation medium comprising all component necessary for the growth of the one or more microorganisms producing a fermentation broth comprising the one or more microorganisms, spent substrate components and one or more desired fermentation products. If the yield of the desired fermentation product is sufficient high, part of the fermentation product may precipitate forming crystals or amorphous precipitates in the fermentation broth. This is well known in the art and many fermentation processes, microorganisms, medium components have been disclosed in the art. The invention is not limited by the particular fermentation process as long as the fermentation process provides a fermentation broth comprising a desired fermentation product at least partially in insoluble form in the fermentation broth such as in crystalline or amorphous form.

Methods for fermenting microorganism for producing a desired product where the desired product precipitates during the fermentation has been described in the art e.g. US 6,316,240, WO 03/050274, WO 93/13125 and EP2125865 and any of these methods for fermenting the microorganisms can be used together with the methods of the present invention.

The fermentation process may be any suitable fermentation process providing a fermentation broth comprising a desired fermentation product at least partially in insoluble form, such as a batch fermentation, fed-batch or continuous fermentation.

The present invention may be useful for any fermentation in industrial scale, e.g. for any fermentation having culture media of at least 50 liters, such as at least 500 liters, such as at least 5,000 liters, such as at least 50,000 liters.

The fermentation product is one or more enzyme selected among proteases having at least 80% sequence identity, such as at least 85% sequence identity, such as at least 95% sequence identity, such as at least 96% sequence identity, such as at least 97% sequence identity, such as at least 98% sequence identity, such as at least 99% sequence identity to one of SEQ ID NO: 1- 6, or selected among amylases having at least 80% sequence identity, such as at least 85% sequence identity, such as at least 95% sequence identity, such as at least 96% sequence identity, such as at least 97% sequence identity, such as at least 98% sequence identity, such as at least 99% sequence identity to one of SEQ ID NO: 7-9.

In some embodiments the fermentation product according to the invention is a protease having at least 80%, at least 90%, at least 95%, at least 96%, at least 97%, at least 98% or at least 99% sequence identity to the sequence of SEQ ID NO: 1, SEQ ID NO: 2, SEQ ID NO: 3, SEQ ID NO: 4, SEQ ID NO: 5 or SEQ ID NO: 6.

In some embodiments the fermentation product according to the invention is an amylase having at least 80%, at least 90%, at least 95%, at least 96%, at least 97%, at least 98% or at least 99% sequence identity to the sequence of SEQ ID NO: 7, SEQ ID NO: 8 or SEQ ID NO: 9.

The fermentation product may be obtained from a microorganism. The microorganism may be an organism that naturally produces the fermentation product or it may be generated using recombinant DNA technology where a gene encoding the desired fermentation product is operably linked with suitable control sequences such as promoter, terminator etc., suitable for expressing the gene in the intended host organism, and inserted into a suitable host organism.

The microorganism may be a prokaryot or an eukaryot.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any *Bacillus* cell including, but not limited to, *Bacillus alkalophilus, Bacillus altitudinis, Bacillus amyloliquefaciens, B. amyloliquefaciens* subsp. *plantarum, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus methylotrophicus, Bacillus pumilus, Bacillus safensis, Bacillus stearothermophilus, Bacillus subtilis,* and *Bacillus thuringiensis* cells.

The bacterial host cell may also be any *Streptococcus* cell including, but not limited to, *Streptococcus equisimilis, Streptococcus pyogenes, Streptococcus uberis,* and *Streptococcus equi subsp. Zooepidemicus* cells.

The bacterial host cell may also be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a *Bacillus* cell may be effected by protoplast transformation (see, *e.g.,* Chang and Cohen, 1979, Mol. Gen. Genet. 168: 111-115), competent cell transformation (see, *e.g.,* Young and Spizizen, 1961, J. Bacteriol. 81: 823-829, or Dubnau and Davidoff-Abelson, 1971, J. Mol. Biol. 56: 209-221), electroporation (see, *e.g.,* Shigekawa and Dower, 1988, Biotechniques 6: 742-751), or conjugation (see, *e.g.,* Koehler and Thorne, 1987, J. Bacteriol. 169: 5271-5278). The introduction of DNA into an *E. coli* cell may be effected by protoplast transformation (see, e.g., Hanahan, 1983, J. Mol. Biol. 166: 557-580) or electroporation (see, *e.g.,* Dower et al., 1988, Nucleic Acids Res. 16: 6127-6145). The introduction of DNA into a *Streptomyces* cell may be effected by protoplast transformation, electroporation (see, *e.g.,* Gong et al., 2004, Folia Microbiol. (Praha) 49: 399-405), conjugation (see, *e.g.,* Mazodier et al., 1989, J. Bacteriol. 171: 3583-3585), or transduction (see, *e.g.,* Burke et al., 2001, Proc. Natl. Acad. Sci. USA 98: 6289-6294). The introduction of DNA into a *Pseudomonas* cell may be effected by electroporation (see, *e.g.,* Choi et al., 2006, J. Microbiol. Methods 64: 391-397) or conjugation (see, *e.g*., Pinedo and Smets, 2005, Appl. Environ. Microbiol. 71: 51-57). The introduction of DNA into a *Streptococcus* cell may be effected by natural competence (see, *e.g.,* Perry and Kuramitsu, 1981, Infect. Immun. 32: 1295-1297), protoplast transformation (see, *e.g.,* Catt and Jollick, 1991, Microbios 68: 189-207), electroporation (see, *e.g.,* Buckley et al., 1999, Appl. Environ. Microbiol. 65: 3800-3804), or conjugation (see, *e.g.,* Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (*Endomycetales*)*,* basidiosporogenous yeast, and yeast belonging to the *Fungi Imperfecti* (*Blastomycetes*)*.* Since the classification of yeast may change in the future, for the purposes of this invention, yeast shall be defined as described in *Biology and Activities of Yeast* (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis, or Yarrowia lipolytica* cell.

The fungal host cell may be a filamentous fungal cell. "Filamentous fungi" include all filamentous forms of the subdivision Eumycota and Oomycota (as defined by Hawksworth *et al.,* 1995, *supra*)*.* The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

### Pretreatments

Before the separation method according to the invention the fermentation broth may be subjected to one or more pretreatment steps, such as dilution, adjusting pH and/or temperature, adding stabilizers capable of preventing further growth of the microorganism, adding inhibitors capable of reducing protease activity and thereby limiting degradation due to proteolytic activity.

The pretreatment may also comprise a lysis step e.g. treating the broth with a chemical lysing agents such as one or more cell wall degrading enzymes e.g. lysozyme(s). Preferred lysis methods include adding lysozyme and/or hydrolysing enzymes to solubilise suspended material as cells or residual fermentation raw materials.

### First separation step

The fermentation broth comprising a desired fermentation product at least partially in solid form is according to the invention separated into at least two fraction in a first separation step, a first fraction comprising a liquid part of the fermentation broth comprising desired product in soluble form as well as other solubles such as salts and remaining soluble nutrients and it may comprise cells and cell debris; and a second fraction comprising the desired product in solid form, cells, cell debris and other insoluble material. The first phase generated in the first separation step may also be called the primary centrate or filtrate. The second phase may also be called the slurry or sludge phase.

Both the first fraction and the second fraction may comprise liquid part of the fermentation broth, cells and cell debris and desired product in solid form, but the ratios are completely different.

The first fraction comprises at least 60 % of the liquid part of the fermentation broth, e.g. at least 70%, e.g. at least 80%, of the fermentation broth, whereas the second fraction comprises the reminder.

The second fraction comprises at least 60% of the desired product in solid form, preferably at least 65%, preferably at least 70%, preferably at least 75%, preferably at least 80%, preferably at least 85%, and most preferred at least 90%.

The cells and cell debris is divided between the first and the second phase and the ratio depends on the particular separation technology used in the first separation step.

The first separation step is basically a separation step where solids are completely or partially separated from the liquid and the first separation step may therefore be performed using any technique known in the art for separating solids from a liquid phase. The first separation step may be done using filtration, decanting or centrifugation or any combination of these, and the separation may be done batch wise or continuously.

Depending on the selected separation technology used in the first separation step, it may be beneficial to add one or more Chemicals for assisting the separation before the separation. For example, one or more flocculants may be added before the first separation process as known in the art. The type and amount of the flocculants are selected based on the properties of the particular selected fermentation broth and will typically be determined using simple routing experimentation. This it known in the art and the selection of type and amount of one or more flocculants to facilitate the separation is completely within the skills of the skilled practitioner. For example may the methods disclosed in WO 2004/001054 be used according to the present invention.

### Solubilization step.

The solubilisation step where desired product in the second phase is solubilized may be performed in any known method for solubilizing solid products, typically involving dilution with water or an aqueous medium, adding chemicals enhancing solubilisation and/or adjusting the pH.

The prior art discloses methods for solubilizing proteins, in particular proteins, in solid form in for example EP 2125865; US 3,316,240 and WO 93/13125 and these methods are also useable according to the present invention.

In one embodiment the solubilisation step is performed by diluting the second phase with water or an aqueous media, optionally adding a solubilisation promoting chemical and reducing the pH.

In this embodiment the solubilisation may be done by diluting the second phase comprising desired product in solid form 100-2000% (w/w) based on the amount of the second phase, adjusting the pH to a pH value below 6.0, such as below 5.5, such as below 5.0, such as below 4.5; adding a solubilisation promoting chemical and mixing, whereby desired product is solubilized. The second phase comprising desired product in solid form is diluted 100-2000% (w/w), preferably 100-1500% (w/w), more preferred 100-1000% (w/w) and most preferred 200-700% (w/w).

The solubilsation promoting chemical may be any chemical promoting the solubilisation process e.g. a divalent salt, i.e. any salt comprising a divalent metal ion and soluble in the sludge phase; typically, a salt of Calcium, Magnesium, Ferric, Zinc salt comprising an anion selected among phosphates, sulphate, nitrate, acetate, chloride. Calcium or Magnesium salts are preferred. The solubilisation promoting chemical may be added at a concentration of 0.01-5% (w/w) based on the diluted second phase, preferably 0.01-1 % (w/w) more preferred in the range of 0.1-0.5% (w/w).

The dilution medium will typically be water or an aqueous medium, such as an ultrafiltrate permeate or a condensate from an evaporation step recycled from another process performed at the same site such as in a subsequent step in the product recovery process.

The pH of the fermentation broth is in this embodiment adjusted to a pH value below pH 6.0, preferably below pH 5.5, in particular to a pH value below 5.0. The pH adjustment may be done before, simultaneously or after the addition of the divalent salt. The pH may be adjusted to a pH value between 2.0 and 5.5; preferably to a pH value between 2.0 and 5.0; more preferably to a pH value between 3.0 and 5.0, and in particular to a pH value between 4.0 and 5.0. It is to be noted that the process steps of the solubilisation step the dilution and the addition of a divalent salt and the pH adjustment may take place simultaneously or in any order. After adding the divalent salt and adjusting the pH, a mixing will take place. The mixing time will depend on the chosen temperature and the crystal morphology and/or structure of the protein in question. More than 80% of the crystals and/or precipitate and/or desired product bound to cell mass/insolubles may be solubilized according to the present invention; preferably more than 85%; more preferably more than 90% and in particular more than 95% of the crystals and/or precipitate and/or desired product bound to cell mass/insolubles may be solubilized

In another embodiment the solubilisation of the desired product in the second phase may be done by diluting the second phase with water or an aqueous solution and adjusting the pH to a high pH value, preferably to a pH value in the range of 9.5 to 13, such as the range of 10 to 13.

In still another embodiment the solubilisation or the desired product in the second phase is done by adding a chemical enhancing solubilisation of the desired product. The chemical enhancing solubilisation is preferably a polyol such as a low molecular weight polyethylene glycol, and the C₂ to C₈ alcohols having at least two OH groups, preferably with only two OH groups; especially preferred is the polyols where two OH groups are present on adjacent carbon atoms in the chain and the C₂-C₈ alcohol is aliphatic and have a straight carbon chain. Preferred polyols include ethylene glycol, propylene glycol, mono-propylene glycol, glycerol, the low molecular weight (about 900 or less) polyethylene glycols, and mixtures thereof.

In some embodiment an extended residence time is inserted after all ingredients and adjustments have been added in order to allow the dissolution of the desired product to take place. Like other chemical process the solubilisation of the desired product take time and a resident period may be beneficial to allow more product to dissolve. In principle the residence time may be extended until all the desired product is dissolved, but often the soluble product is more susceptible to degradation e.g. by proteases from the fermentation broth compared with the precipitated product, so in practice the skilled person often will select a relative short residence time in order to get as much desired product in solution but avoiding too much degradation of the soluble product.

The residence may typically be up to 12 hours, such as in the range of 0-500 minutes, preferably in the range of 15-300 minutes, more preferred in the range of 30-90 minutes.

After the solubilisation step the desired product may be recovered using methods known in the art such as further separation steps removing cells and cell debris and other solids from the mixture, concentration, formulation, drying such as spray drying, granulation etc.

In some applications the mixture is subjected to a lysis step after the solubilisation step such as treatment with a chemical lysing agents such as a cell wall degrading enzymes e.g. lysozyme. Preferred lysis methods include. adding lysozyme or hydrolysis enzymes to solubilise suspended material as cells or residual fermentation raw materials.

The particular selected process will be determined by several different factors such as the intended use of the desired product, available equipment and the amount of cells and cell debris in the second phase.

### Second separation step

In a preferred embodiment the method of the invention further comprises a second separation step removing cells and cell debris from the mixture comprising the solubilized desired product.

After the solubilisation step the mixture is subjected to the second separation step, which is basically a solid/liquid separation process removing solids, such as cells, cell debris and solids originating from the substrate; from the liquid fraction comprising the solubilized desired product. The second separation may be performed using any such equipment and methods known in the art for solid/liquid separations, such as centrifuges, decanters, filtration etc.

Depending on the selected separation technology used in the first separation step, it may be beneficial to add one or more Chemicals for assisting the separation before the separation. For example, one or more flocculants may be added before the first separation process as known in the art. The type and amount of the flocculants are selected based on the properties of the particular selected fermentation broth and will typically be determined using simple routing experimentation. This it known in the art and the selection of type and amount of one or more flocculants to facilitate the separation is completely within the skills of the skilled practitioner.

In one preferred embodiment, part or all of the first phase from the first separation is mixed with the mixture coming from the solubilisation step. In this way the two streams are combined and the desired product, both the part that was in solution in the fermentation broth and consequently contained in the first phase of the first separation step, and the part that was precipitated in the fermentation broth and consequently ended in the second phase of the first separation step, can be further purified and processed together in subsequent downstream operations.

This is particular beneficial for fermentations where the first phase comprises a significant amount of the desired product is solution. By mixing the first phase with the solubilized second phase before or after the second separation step will allow the skilled person to recover all desired product, both the part present in solid form in the second phase and the part present in solution in the first phase, together in downstream processes.

The mixing of the first phase of the first separation step and the stream from the solubilisation step comprising the solubilized desired product can take place either before or after the second separation step. If the first separation is performed in a way where the first phase comprises a significant amount of cell and cell debris is it preferred to add the first phase to the solubilized second phase before the second separation step so all cells and cell debris can be removed in the second separation step. If the first separation step is performed in a way so the first phase comprises no or only very minor amounts of the cells and cell debris; e.g. less than 1 % of the total amount; the first phase may be added to the solubilized second phase either before or after the second separation step.

The skilled person will appreciate that the temperature may influence the process in various ways; the temperature may influence the solubilisation kinetics e.g. the solubilisation proceeds faster at a higher temperature; the temperature may also influence the stability of the desired product e.g. may the stability of a desired product decrease when the temperature rises. This means that for a given recovery process, the skilled person will have to optimize the temperature in order to obtain the best possible recovery process, and further it means that the temperature that is optimal for one fermentation product may not be optimal for another fermentation product. This is all known to the skilled person and can be done using typical routine experiments. The process of the invention is typically performed at a temperature in the range of 0-70°C, e.g. in the range of 10-50°C, often in the range of 15-45°C.

The method according to the invention has the benefit compared with prior art processes such as the method disclosed in EP 2 125 865 that the volume needed for solubilizing the desired product is significantly lower. Without wishing to be limited by any theory it is believed that this is because a substantial part of the salts present during fermentation where the precipitation took place has been removed with the first phase and is therefore absent during the solubilisation process. This has the benefit that the water consumption and /or chemicals enhancing solubilisation is significantly lower which has a number of significant additional benefits in industrial production scale operations, in addition to the cost of water and chemicals enhancing solubilisation; such as a reduced volume of waste water that need to be treated and further, the capacity costs for handling large volumes, equipment for downstream processing is reduced because the volumes that need to be treated are significantly lower.

Further, the solubilisation of a precipitated product according to the invention may also proceed faster that the solubilisation of the same product using a method according to prior art, giving the advantage of a higher throughput using the method according to the invention and consequently a lower demand for holding capacity.

### Subsequent downstream operations

The resulting desired product may be further isolated by methods known in the art. For example, the desired product may be recovered by conventional procedures including, but not limited to, further filtration, e.g., for removing any germs; ultra-filtration and micro-filtration, centrifugation, extraction, spray-drying, evaporation, precipitation or crystallization, hydrolysis (e.g. lysozyme or other) or other mechanical suspension degradation

### EXAMPLES

### Materials and Methods

Fermentation Broths for the examples below were provided from Novozymes A/S, Kalundborg, Denmark. The fermentation broths were prepared by inoculating a *Bacillus licheniformis* strain transformed with a gene encoding the desired protease operationally connected with a promoter and regulatory sequences for expressing said gene; in an industrial fermenter in a fed-batch fermentation process. The fermentation process was terminated at the desired product concentration and the desired product was present in precipitated form at that time. By visual inspection using a microscope it could be clearly seen that the fermentation broth comprised crystalline material in addition to other solids including cells and cell debris.

In the examples below, the amount of solubilized product is defined as the ratio of product detected in the supernatant of a sample that has been subjected to a Relative Centrifugal Force of 2333 for 5 minutes, and the amount of product detected in an uncentrifuged sample.

Full dissolution is defined as occurring when the supernatant product concentration is within the measurement uncertainty of the concentration measured in an uncentrifuged sample. This value can be measured directly or calculated by extrapolation from multiple measurements.

The level of dilution of the fermentation broth in the given examples is used to reduce the concentration of components that are detrimental to solubility. The criteria for the level of dilution used in these examples is that it is high enough to solubilize all the product, but not in excess, to minimize water usage and process volumes.

The residence time for dissolution given in these examples is defined by process or experimental convenience and does not necessarily ensure full dissolution

### Example 1 - Recovery of Savinase

A fermentation broth from an industrial fermentation producing the protease having the amino acid sequence of SEQ ID NO: 1 (Savinase) was used in this example.

### A - Recovery according to prior art

Fermentation broth holding crystallized product enzyme was diluted 10 times with water (9L water added per kg Fermentation broth). Poly Aluminium Chloride and 34 % (w/w) CaCl₂ was added (25 ml and 70 ml per kg Fermentation broth respectively) and the pH of the solution was adjusted to pH 4.2-4.6 using acetic acid. The mixture was given a residence time of 90 minutes at 41°C whereby more that 80% of the product crystals were dissolved. The total volume was a little more than 10 times the initial volume of Fermentation broth.

This solution was then processed by a standard primary separation method: flocculation by cationic and anionic polymers and centrifugation to separate the solids from the liquid holding the dissolved product.

The liquid part was then processed further downstream in a series of steps for final product purification according to company specifications.

### B - Recovery according to the invention

Fermentation broth holding crystallized product enzyme was diluted 3 times with water (2 kg water added per kg Fermentation broth) and separated according to the invention by centrifugation into a first phase (liquid phase) holding some cells and debris and a second phase (slurry) holding almost all the crystallized enzyme and some cells and debris. The separation was performed using a Custom designed centrifuge as further described in US 8889395B2. The first and second phases were both collected in separately. Approx. 0.75 kg second phase and 2.25 kg first phase was produced per kg Fermentation broth.

The second phase holding almost all the crystallized product was then diluted 12-13 times (11-12 kg water per kg second phase). Poly Aluminium Chloride and 34 % (w/w) CaCl₂ was added (2.3 ml and 23 ml per kg second phase respectively) and the solution was pH adjusted to pH 4.2-4.6 using acetic acid. The mixture was given a residence time of 90 minutes at 41°C whereby more that 80% of the crystals were dissolved.

The first phase from the first separation was then added to the diluted and solubilized second phase ending with a total volume of only a little more than 8 times the initial volume of culture broth. This clearly demonstrates reduced water consumption and process volumes using the method according to the invention.

This solution was then processed by a standard primary separation method: flocculation by cationic and anionic polymers and centrifugation to separate the solids from the liquid holding the dissolved product.

The liquid part was then processed further downstream in a series of steps for final product purification according to company specifications.

### Example 2 - Savinase variant

A fermentation broth from an industrial fermentation producing a variant of the polypeptide having the amino acid sequence of SEQ ID NO: 1 with the substitutions: Y176A +R170S+A194P was used in this example. The product has precipitated and crystal were abundant in the fermentation broth.

### A - Recovery according to prior art.

Fermentation broth holding crystalized enzyme was diluted 14 times with water (13 kg water added per kg Fermentation broth). Poly Aluminium Chloride and 34 % (w/w) CaCl₂ was added (25 ml and 80 ml per kg Fermentation broth respectively) and the solution was pH adjusted to pH 4.2-4.6 using acetic acid. The mixture was given a residence time of 90 minutes at 42°C whereby more that 70% of the product crystals were dissolved. The total volume was a little more than 14 times the initial volume of Fermentation broth.

This solution was then processed by a standard primary separation method: flocculation by cationic and anionic polymers and centrifugation to separate the solids from the liquid holding the dissolved product.

The liquid part was then processed further downstream in a series of steps for final product purification according to company specifications.

### B - Recovery according to the invention.

Fermentation broth holding crystallized product enzyme was diluted 3 times with water (2 kg water added per kg Fermentation broth) and separated according to the invention by centrifugation into a first phase (liquid phase) holding some cells and debris and a second phase (slurry) holding almost all the crystallized enzyme and some cells and debris. The separation was performed using a Custom designed centrifuge as further described in US 8889395B2. Approx. 0.5 kg second phase and 2 kg first phase was produced per kg Fermentation broth.

The second phase holding almost all the crystallized product was then diluted 14 times (13 kg water per kg second phase). Poly Aluminium Chloride and 34 % (w/w) CaCl₂ was added (11.4 ml and 11 ml per kg second phase) and the solution was pH adjusted to pH 4.2-4.6 using acetic acid. The mixture was given a residence time of 90 minutes at 42°C whereby more that 70% of the crystals were dissolved.

The total volume was only a little more than 7 times the initial volume of culture broth and clearly demonstrates reduced water consumption and process volumes using the method according to the invention.

This solution was then processed by a standard primary separation method: flocculation by cationic and anionic polymers and centrifugation to separate the solids from the liquid holding the dissolved product.

The liquid part was then processed further downstream in a series of steps for final product purification according to company specifications.

### Example 3 - Recovery of Savinase via drum filtration.

A fermentation broth from an industrial fermentation producing the protease having the amino acid sequence of SEQ ID NO: 1 (Savinase) was used in this example.

### A - recovery according to prior art

In this example, the fermentation broth holding crystallized product enzyme was diluted 10 times with tap water (9 kg water added per kg Fermentation broth), and then adjusted with 60ml 34 % (w/w) CaCl₂ solution and 50ml Poly Aluminium Chloride per kg fermentation broth. The pH was adjusted to pH 4.5 using acetic acid. The mixture was given a residence time 90 minutes at 42°C whereby more that 80% of the crystals were dissolved. The total volume was a little more than 10 times the initial volume of Fermentation broth.

### B - recovery according to the invention

Fermentation broth holding crystallized product enzyme was diluted 3 times with tap water (2 kg water added per kg fermentation broth) and then adjusted with 20.0 ml Poly Aluminium Chloride per kg fermentation broth and pH was adjusted to pH 4.5 using acetic acid. 200 ml poly cationic polymer per kg fermented broth was added to aid separation by drum filtration into a first phase containing solubilized material and a second phase holding all the crystallized enzyme, cells and debris. The first and second phases were both collected in separate holding tank. Approximately 1 kg of second phase and 2 kg first phase was produced per kg Fermentation broth.

The second phase holding almost all the crystallized product, cells and cell debris was then diluted 3 times with tap water (2 kg water per kg second phase). A CaCl₂ (34 % (w/w)) solution was added (18mls per kg second phase) and pH was adjusted to pH 4.5 using acetic acid. The mixture was left standing in 60 minutes whereby more that 80% of the crystals were dissolved.

The first phase from the first separation was then added to the diluted and solubilized second phase ending with a total volume of a little more than 5 times the initial volume of the Fermentation broth and clearly demonstrating reduced water consumption and process volumes using the method according to the invention.

### Example 4 - Recovery of amylase via centrifugation.

A fermentation broth from an industrial fermentation producing an amylase variant prepared in Example 8 of WO 2001/066721 was used in this example.

### A - recovery according to prior art

In this example, the fermentation broth holding crystallized product enzyme was diluted 20 times with tap water (19 kg water added per kg Fermentation broth), and then adjusted with 95 ml 34 % (w/w) CaCl₂ solution and 30ml Poly Aluminum Chloride per kg fermentation broth. The pH was adjusted to pH 5.0 using acetic acid. The mixture was given a residence time 20 minutes at 50°C whereby more that 85% of the crystals were dissolved. The total volume was a little more than 20 times the initial volume of Fermentation broth.

### B - recovery according to the invention

Fermentation broth holding crystallized product enzyme was centrifuged for 5 mins at 2666 RCF. The upper half of the volume containing biomass and less than 5% of the product enzyme was removed and discarded. The remaining lower fraction containing more than 95% of the product enzyme was diluted 30 times with tap water (the equivalent of 29 kg water added per kg lower fraction) and then adjusted with 70 ml 34 % (w/w) CaCl₂ solution and 30,0 ml Poly Aluminum Chloride per kg fermentation broth and pH was adjusted to pH 5,0 using acetic acid. The mixture was given a residence time 20 minutes at 50°C whereby more that 90% of the crystals were dissolved. The total volume was a little more than 15 times the initial volume of Fermentation broth, clearly demonstrating reduced water consumption and process volumes using the method according to the invention.

### Example 5 - Recovery of fungal based Muramidase

Fermentation Broths for this example were provided from Pilot plant, Novozymes A/S, Bagsværd, Denmark. The fermentation broth was prepared by inoculating a fungal (*Trichoderma reesei*) strain transformed with a gene encoding the muramidase (SEQ ID NO: 4 in WO 2013/076253) operationally connected with a promoter and regulatory sequences for expressing said gene; in a large pilot-scale fermenter in a fed-batch fermentation process. By visual inspection using a microscope it could be clearly seen that the fermentation broth comprised crystalline material in addition to other solids including cells and cell debris.

The amount of solubilized product is in this example defined as the ratio of product detected in the supernatant of a sample that has been subjected to a Relative Centrifugal Force of 2333 for 5 minutes, and the amount of product detected in an uncentrifuged sample.

Full dissolution is defined as occurring when the supernatant product concentration is within the measurement uncertainty of the concentration measured in an uncentrifuged sample. This value can be measured directly or calculated by extrapolation from multiple measurements.

The level of dilution of the fermentation broth in the given examples is used to reduce the concentration of components that are detrimental to solubility. The criteria for the level of dilution used in these examples is that it is high enough to solubilize all the product, but not in excess, to minimize water usage and process volumes.

The residence time for dissolution given in these examples is defined by process or experimental convenience and does not necessarily ensure full dissolution.

### A - Recovery according to prior art

Fermentation broth holding crystalized enzyme was diluted 7 times with process water (6 kg water added per kg Fermentation broth). Poly Aluminium Chloride was added (15 ml per kg Fermentation broth) and the solution was pH adjusted using phosphoric acid. The mixture was given a residence time of 120 minutes at 25°C whereby more that 75% of the product crystals were dissolved. The total volume was a little more than 7 times the initial volume of Fermentation broth.

This solution was then processed by a standard primary separation method: flocculation by cationic and anionic polymers and centrifugation to separate the solids from the liquid holding the dissolved product.

The liquid part was then processed further downstream in a series of steps for final product purification according to company specifications.

### B - recovery according to the invention

Fermentation broth holding crystallized product enzyme was diluted 2,5 times with process water (1,5 kg water added per kg Fermentation broth) and separated according to the invention by centrifugation into a first phase (liquid phase) holding some cells and debris and a second phase (slurry) holding almost all the crystallized enzyme and some cells and debris. The separation was performed using a SB7 (Westfalia) discharge centrifuge. The first and second phases were both collected separately. Approx. 0.55 kg second phase and 1,95 kg first phase was produced per kg Fermentation broth.

The second phase holding almost all the crystallized product was then treated by exactly the same method as regular Culture broth; diluted 7 times (6 kg process water per kg second phase). Poly Aluminium Chloride was added (15 ml per kg second phase) and the solution was pH adjusted using phosphoric acid. This mixture was just given a residence time of 60 minutes at 25°C whereby more that 85% of the crystals were dissolved.

The first phase from the first separation was then added to the diluted and solubilized second phase ending with a total volume of approx. 6 times the initial volume of culture broth. This demonstrates reduced water consumption and process volumes as well as shorter dissolution time (higher dissolved product yield) using the method according to the invention.

This solution was then processed by a standard primary separation method: flocculation by cationic and anionic polymers and centrifugation to separate the solids from the liquid holding the dissolved product.

The liquid part was then processed further downstream in a series of steps for final product purification according to company specifications.

The dissolution curves for the culture broth (CB) according to part A, and the slurry found in the second phase according to part B are shown in figure 1, and it shown clearly that the dissolution proceeded faster using the method according to the present invention, compared with the traditional method. The figure also shows that a higher dissolution was obtained after 60 minutes according to the invention compared with after 120 minutes using the procedure of the prior art.

## Claims

1. A method for recovering one or more enzyme from a fermentation broth, where the one or more enzyme is present in precipitated form in the fermentation broth, the method comprising the steps of:
a) a first separation step separating the fermentation broth in a first phase and a second phase, wherein the first phase comprises supernatant, the one or more enzyme in soluble form and optionally cells and cell debris, and the second phase comprises the one or more enzyme in precipitated form, cells and cell debris; and
b) a solubilization step where the one or more enzyme in precipitated form in the second phase is solubilized,
wherein the one or more enzyme is selected among proteases having at least 80% sequence identity, such as at least 85% sequence identity, such as at least 95% sequence identity, such as at least 96% sequence identity, such as at least 97% sequence identity, such as at least 98% sequence identity, such as at least 99% sequence identity to one of SEQ ID NO: 1 - 6, or selected among amylases having at least 80% sequence identity, such as at least 85% sequence identity, such as at least 95% sequence identity, such as at least 96% sequence identity, such as at least 97% sequence identity, such as at least 98% sequence identity, such as at least 99% sequence identity to one of SEQ ID NO: 7-9.

2. The method according to claim 1, further comprising a second separation step where the solubilized one or more enzyme from step b) is separated from the cells and/or cell debris.

3. The method according to claim 1 or 2, wherein the first phase comprises at least 60 % of the liquid part of the fermentation broth, e.g. at least 70%, e.g. at least 80% of the fermentation broth.

4. The method according to any of the claims 1-3, wherein the second fraction comprises at least 60% of the one or more enzyme in solid form, preferably at least 65%, e.g. at least 70%, e.g. at least 75%, e.g. at least 80%, e.g. at least 85%, and e.g. at least 99% of the one or more enzyme in solid form.

5. The method according to any of the preceding claims, wherein the first separation step is performed using centrifugation or filtration.

6. The method according to any of the preceding claims, wherein the solubilization step comprises:
i. Diluting the second phase 100-2000% (w/w) in water or an aqueous medium;
ii. adding a divalent salt; and
iii. adjusting the pH to a pH value below 6.0

7. The method according to claim 6, wherein the second phase is diluted 100-2000% (w/w), preferably 100-1500% (w/w), preferably 100-1000% (w/w) and most preferred 200-700% (w/w).

8. The method according to any of the claims 6 - 7, wherein the divalent salt is selected among Calcium, Magnesium, Ferrous and Zinc salts comprising an anion selected among phosphates, sulphate, nitrate, acetate and chloride and is added in an amount of 0.01-5% (w/w) based on the diluted second phase, preferably 0.01-1% (w/w) more preferred in the range of 0.1-0.5% (w/w).

9. The method according to any of the claims 6 - 8, wherein the pH is adjusted to a pH value below pH 6.0, preferably below pH 5.5, in particular to a pH value below 5.0, wherein the pH adjustment may be done before, simultaneously or after the addition of the divalent salt, and wherein the pH may be adjusted to a pH value between 2.0 and 5.5; preferably to a pH value between 2.0 and 5.0; more preferably to a pH value between 3.0 and 5.0, and in particular to a pH value between 4.0 and 5.0.

10. The method according to any of the claims 1 - 5, wherein the solubilisation step is done by diluting the second phase with water or an aqueous solution and adjusting the pH to a pH value above 9.5, such as to a pH value in the range of 9.5 to 13, e.g. to a pH value in the range of 10 to 13.

11. The method according to any of the claims 1 - 5, wherein the solubilisation step is done by adding a chemical enhancing the solubilisation of the one or more enzyme.

12. The method according to claim 11, wherein the chemical enhancing the solubilisation of the one or more enzyme is a polyol such as a low molecular weight polyethylene glycol or C₂ to C₈ alcohols having at least two OH groups, preferably with only two OH groups; especially preferred is the polyols where two OH groups are present on adjacent carbon atoms in the chain and the C₂-C₈ alcohol is aliphatic and have a straight carbon chain.

13. The method according to any of the preceding claims where the first phase from the first separation step is partly or completely added to the solubilized second phase.

14. The method according to any of the previous claims comprising a pretreatment step before the first separation step selected among: dilution; adjusting pH and/or temperature, adding one or more stabilizers, adding one or more protease inhibitors.

15. The method according to any of the previous claims comprising one or more downstream operations after the second separation step, selected among: ultrafiltration, evaporation, concentration, stabilization, crystallization, spray drying and granulation.

## Patentansprüche

1. Verfahren zur Rückgewinnung eines oder mehrerer Enzyme aus einer Fermentationsbrühe, wobei das eine oder die mehreren Enzyme in der Fermentationsbrühe in ausgefällter Form vorliegen, wobei das Verfahren die folgenden Schritte umfasst:
a) einen ersten Trennschritt, bei dem die Fermentationsbrühe in eine erste Phase und eine zweite Phase getrennt wird, wobei die erste Phase Überstand, das eine oder die mehreren Enzyme in löslicher Form und gegebenenfalls Zellen und Zelltrümmer umfasst und die zweite Phase das eine oder die mehreren Enzyme in ausgefällter Form, Zellen und Zelltrümmer umfasst, und
b) einen Solubilisierungsschritt, bei dem das eine oder die mehreren Enzyme in ausgefällter Form in der zweiten Phase solubilisiert werden,
wobei das eine oder die mehreren Enzyme aus Proteasen mit mindestens 80% Sequenzidentität, wie mindestens 85% Sequenzidentität, wie mindestens 95% Sequenzidentität, wie mindestens 96% Sequenzidentität, wie mindestens 97% Sequenzidentität, wie mindestens 98% Sequenzidentität, wie mindestens 99% Sequenzidentität mit einer der SEQ ID NO: 1-6 ausgewählt sind oder aus Amylase mit mindestens 80% Sequenzidentität, wie mindestens 85% Sequenzidentität, wie mindestens 95% Sequenzidentität, wie mindestens 96% Sequenzidentität, wie mindestens 97% Sequenzidentität, wie mindestens 98% Sequenzidentität, wie mindestens 99% Sequenzidentität mit einer der SEQ ID NO: 7-9 ausgewählt sind.

2. Verfahren nach Anspruch 1, weiterhin umfassend einen zweiten Trennschritt, bei dem das eine oder die mehreren solubilisierten Enzyme aus Schritt b) von den Zellen und/oder Zelltrümmern abgetrennt werden.

3. Verfahren nach Anspruch 1 oder 2, wobei die erste Phase mindestens 60% des flüssigen Teils der Fermentationsbrühe, z. B. mindestens 70%, z. B. mindestens 80% der Fermentationsbrühe umfasst.

4. Verfahren nach einem der Ansprüche 1-3, wobei die zweite Fraktion mindestens 60% des einen oder der mehreren Enzyme in fester Form, vorzugsweise mindestens 65%, z. B. mindestens 70%, z. B. mindestens 75%, z. B. mindestens 80%, z. B. mindestens 85% und z. B. mindestens 99% des einen oder der mehreren Enzyme in fester Form umfasst.

5. Verfahren nach einem der vorhergehenden Ansprüche, wobei der erste Trennschritt unter Anwendung von Zentrifugation oder Filtration durchgeführt wird.

6. Verfahren nach einem der vorhergehenden Ansprüche, wobei der Solubilisierungsschritt Folgendes umfasst:
i. das Verdünnen der zweiten Phase 100-2000% (w/w) mit Wasser oder einem wässrigen Medium,
ii. die Zugabe eines zweiwertigen Salzes und
iii. das Einstellen des pH-Wertes auf einen pH-Wert unter 6,0.

7. Verfahren nach Anspruch 6, wobei die zweite Phase 100-2000% (w/w), vorzugsweise 100-1500% (w/w), vorzugsweise 100-1000% (w/w) und am meisten bevorzugt 200-700% (w/w) verdünnt wird.

8. Verfahren nach einem der Ansprüche 6-7, wobei das zweiwertige Salz aus Calcium-, Magnesium-, Eisen- und Zinksalzen mit einem aus Phosphaten, Sulphat, Nitrat, Acetat und Chlorid ausgewählten Anion ausgewählt ist und in einer Menge von 0,01-5% (w/w), bezogen auf die verdünnte zweite Phase, vorzugsweise 0,01-1% (w/w), besonders bevorzugt im Bereich von 0,1-0,5% (w/w), zugesetzt wird.

9. Verfahren nach einem der Ansprüche 6-8, wobei der pH-Wert auf einen pH-Wert unterhalb eines pH-Wertes unter pH 6,0, vorzugsweise unter pH 5,5, insbesondere auf einen pH-Wert unter 5,0, eingestellt wird und wobei die Einstellung des pH-Wertes vor, während oder nach der Zugabe des zweiwertigen Salzes erfolgen kann, und wobei der pH-Wert auf einen pH-Wert zwischen 2,0 und 5,5, vorzugsweise einen pH-Wert zwischen 2,0 und 5,0, besonders bevorzugt einen pH-Wert zwischen 3,0 und 5,0 und insbesondere auf einen pH-Wert zwischen 4,0 und 5,0 eingestellt werden kann.

10. Verfahren nach einem der Ansprüche 1-5, wobei der Solubilisierungsschritt durch Verdünnen der zweiten Phase mit Wasser oder einer wässrigen Lösung und Einstellen des pH-Wertes auf einen pH-Wert über 9,5, wie einen pH-Wert im Bereich von 9,5 bis 13, z. B. auf einen pH-Wert im Bereich von 10 bis 13, erfolgt.

11. Verfahren nach einem der Ansprüche 1-5, wobei der Solubilisierungsschritt durch Zugabe einer die Solubilisierung des einen oder der mehreren Enzyme verbessernden Chemikalie erfolgt.

12. Verfahren nach Anspruch 11, wobei es sich bei der die Solubilisierung des einen oder der mehreren Enzyme verbessernden Chemikalie um ein Polyol wie ein niedermolekulares Polyethylenglykol oder C₂- bis C₈-Alkohole mit mindestens zwei OH-Gruppen, bevorzugt mit nur zwei OH-Gruppen, handelt, wobei die Polyole besonders bevorzugt sind, bei denen zwei OH-Gruppen an benachbarten Kohlenstoffatomen in der Kette stehen und der C₂-C₈-Alkohol aliphatisch und geradkettig ist.

13. Verfahren nach einem der vorhergehenden Ansprüche, bei dem man die erste Phase aus dem ersten Trennschritt oder einen Teil davon zur solubilisierten zweiten Phase gibt.

14. Verfahren nach einem der vorhergehenden Ansprüche, umfassend einen Vorbehandlungsschritt vor dem ersten Trennschritt ausgewählt aus: Verdünnen, Einstellen des pH-Wertes und/oder der Temperatur, Zugabe eines oder mehrerer Stabilisatoren, Zugabe eines oder mehrerer Proteasehemmer.

15. Verfahren nach einem der vorhergehenden Ansprüche, umfassend einen oder mehrere Arbeitsschritte stromabwärts vom zweiten Trennschritt, ausgewählt aus: Ultrafiltration, Eindampfen, Einengen, Stabilisieren, Kristallisieren, Sprühtrocknen und Granulieren.

## Revendications

1. Procédé pour la récupération d'une ou plusieurs enzymes à partir d'un bouillon de fermentation, dans lequel l'enzyme ou les enzymes sont présentes sous forme précipitée dans le bouillon de fermentation, le procédé comprenant les étapes de :
a) une première étape de séparation séparant le bouillon de fermentation en une première phase et une deuxième phase, la première phase comprenant un surnageant, l'enzyme ou les enzymes sous forme soluble et éventuellement des cellules et des débris cellulaires, et la deuxième phase comprenant l'enzyme ou les enzymes sous forme précipitée, des cellules et des débris cellulaires ; et
b) une étape de solubilisation dans laquelle l'enzyme ou les enzymes sous forme précipitée dans la deuxième phase sont solubilisées,
l'enzyme ou les enzymes étant choisies parmi des protéases possédant au moins 80 % d'identité de séquence, tel qu'au moins 85 % d'identité de séquence, tel qu'au moins 95 % d'identité de séquence, tel qu'au moins 96 % d'identité de séquence, tel qu'au moins 97 % d'identité de séquence, tel qu'au moins 98 % d'identité de séquence, tel qu'au moins 99 % d'identité de séquence par rapport à l'une parmi les SEQ ID N° : 1 à 6, ou choisies parmi des amylases possédant au moins 80 % d'identité de séquence, tel qu'au moins 85 % d'identité de séquence, tel qu'au moins 95 % d'identité de séquence, tel qu'au moins 96 % d'identité de séquence, tel qu'au moins 97 % d'identité de séquence, tel qu'au moins 98 % d'identité de séquence, tel qu'au moins 99 % d'identité de séquence par rapport à l'une parmi les SEQ ID N° : 7 à 9.

2. Procédé selon la revendication 1, comprenant en outre une deuxième étape de séparation dans laquelle l'enzyme ou les enzymes solubilisées de l'étape b) sont séparées des cellules et/ou des débris cellulaires.

3. Procédé selon la revendication 1 ou 2, la première phase comprenant au moins 60 % de la partie liquide du bouillon de fermentation, par ex. au moins 70 %, par ex. au moins 80 % du bouillon de fermentation.

4. Procédé selon l'une quelconque des revendications 1 à 3, la deuxième fraction comprenant au moins 60 % de l'enzyme ou des enzymes sous forme solide, préférablement au moins 65 %, par ex. au moins 70 %, par ex. au moins 75 %, par ex. au moins 80 %, par ex. au moins 85 %, et par ex. au moins 99 % de l'enzyme ou des enzymes sous forme solide.

5. Procédé selon l'une quelconque des revendications précédentes, la première étape de séparation étant réalisée à l'aide d'une centrifugation ou d'une filtration.

6. Procédé selon l'une quelconque des revendications précédentes, l'étape de solubilisation comprenant :
i. la dilution de la deuxième phase de 100 à 2 000 % (p/p) dans de l'eau ou dans un milieu aqueux ;
ii. l'ajout d'un sel divalent ; et
iii. l'ajustement du pH à une valeur de pH inférieure à 6,0.

7. Procédé selon la revendication 6, la deuxième phase étant diluée de 100 à 2 000 % (p/p), préférablement 100 à 1 500 % (p/p), préférablement 100 à 1 000 % (p/p) et de la manière la plus préférée de 200 à 700 % (p/p).

8. Procédé selon l'une quelconque des revendications 6 et 7, le sel divalent étant choisi parmi des sels de Calcium, de Magnésium, Ferreux et de Zinc comprenant un anion choisi parmi des phosphates, un sulfate, un nitrate, un acétate et un chlorure et étant ajouté en une quantité de 0,01 à 5 % (p/p) sur la base de la deuxième phase diluée, préférablement 0,01 à 1 % (p/p), de manière plus préférée dans la plage de 0,1 à 0,5 % (p/p).

9. Procédé selon l'une quelconque des revendications 6 à 8, le pH étant ajusté à une valeur de pH inférieure à une valeur de pH inférieure à pH 6,0, préférablement inférieure à pH 5,5, en particulier à une valeur de pH inférieure à 5,0., l'ajustement du pH pouvant être réalisé avant, simultanément ou après l'ajout du sel divalent, et le pH pouvant être ajusté à une valeur de pH comprise entre 2,0 et 5,5 ; préférablement à une valeur de pH comprise entre 2,0 et 5,0 ; plus préférablement à une valeur de pH comprise entre 3,0 et 5,0, et en particulier à une valeur de pH comprise entre 4,0 et 5,0.

10. Procédé selon l'une quelconque des revendications 1 à 5, l'étape de solubilisation étant réalisée en diluant la deuxième phase avec de l'eau ou une solution aqueuse et en ajustant le pH à une valeur de pH supérieure à 9,5, tel qu'à une valeur de pH dans la plage de 9,5 à 13, par ex. à une valeur de pH dans la plage de 10 à 13.

11. Procédé selon l'une quelconque des revendications 1 à 5, l'étape de solubilisation étant réalisée en ajoutant un composé chimique augmentant la solubilisation de l'enzyme ou des enzymes.

12. Procédé selon la revendication 11, le composé chimique augmentant la solubilisation de l'enzyme ou des enzymes étant un polyol tel qu'un polyéthylène glycol à bas poids moléculaire ou des alcools en C₂ à C₈ possédant au moins deux groupes OH, préférablement comportant seulement deux groupes OH ; notamment préférés sont les polyols dans lesquels deux groupes OH sont présents sur des atomes de carbone adjacents dans la chaîne et l'alcool en C₂ à C₈ étant aliphatique et possédant une chaîne carbonée linéaire.

13. Procédé selon l'une quelconque des revendications précédentes dans lequel la première phase de la première étape de séparation est partiellement ou complètement ajoutée à la deuxième phase solubilisée.

14. Procédé selon l'une quelconque des revendications précédentes comprenant une étape de prétraitement avant la première étape de séparation choisie parmi : une dilution ; un ajustement du pH et/ou de la température, l'ajout d'un ou plusieurs stabilisants, l'ajout d'un ou plusieurs inhibiteurs de protéases.

15. Procédé selon l'une quelconque des revendications précédentes comprenant une ou plusieurs opérations en aval après la deuxième étape de séparation, choisies parmi : une ultrafiltration, une évaporation, une concentration, une stabilisation, une cristallisation, un séchage par pulvérisation et une granulation.


### SEQUENCE LISTING

<110> Novozymes A/S
<120> Recovery process
<130> 14385-WO-PCT
<160> 9
<170> PatentIn version 3.5
<210> 1
   <211> 269
   <212> PRT
   <213> Bacillus lentus
<400> 1
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Gly Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 2
   <211> 275
   <212> PRT
   <213> Bacillus amyloliquefaciens
<400> 2
<210> 3
   <211> 274
   <212> PRT
   <213> Bacillus sp.
<400> 3
   Ala Gln Thr Val Pro Tyr Gly Ile Pro Leu Ile Lys Ala Asp Lys Val
   1               5                   10                  15
   Gln Ala Gln Gly Phe Lys Gly Ala Asn Val Lys Val Ala Val Leu Asp
               20                  25                  30
   Thr Gly Ile Gln Ala Ser His Pro Asp Leu Asn Val Val Gly Gly Ala
           35                  40                  45
   Ser Phe Val Ala Gly Glu Ala Tyr Asn Thr Asp Gly Asn Gly His Gly
       50                  55                  60
   Thr His Val Ala Gly Thr Val Ala Ala Leu Asp Asn Thr Thr Gly Val
   65                  70                  75                  80
   Leu Gly Val Ala Pro Ser Val Ser Leu Tyr Ala Val Lys Val Leu Asn
                   85                  90                  95
   Ser Ser Gly Ser Gly Ser Tyr Ser Gly Ile Val Ser Gly Ile Glu Trp
               100                 105                 110
   Ala Thr Thr Asn Gly Met Asp Val Ile Asn Met Ser Leu Gly Gly Ala
           115                 120                 125
   Ser Gly Ser Thr Ala Met Lys Gln Ala Val Asp Asn Ala Tyr Ala Arg
       130                 135                 140
   Gly Val Val Val Val Ala Ala Ala Gly Asn Ser Gly Ser Ser Gly Asn
   145                 150                 155                 160
   Thr Asn Thr Ile Gly Tyr Pro Ala Lys Tyr Asp Ser Val Ile Ala Val
                   165                 170                 175
   Gly Ala Val Asp Ser Asn Ser Asn Arg Ala Ser Phe Ser Ser Val Gly
               180                 185                 190
   Ala Glu Leu Glu Val Met Ala Pro Gly Ala Gly Val Tyr Ser Thr Tyr
           195                 200                 205
   Pro Thr Asn Thr Tyr Ala Thr Leu Asn Gly Thr Ser Met Ala Ser Pro
       210                 215                 220
   His Val Ala Gly Ala Ala Ala Leu Ile Leu Ser Lys His Pro Asn Leu
   225                 230                 235                 240
   Ser Ala Ser Gln Val Arg Asn Arg Leu Ser Ser Thr Ala Thr Tyr Leu
                   245                 250                 255
   Gly Ser Ser Phe Tyr Tyr Gly Lys Gly Leu Ile Asn Val Glu Ala Ala
               260                 265                 270
   Ala Gln
<210> 4
   <211> 311
   <212> PRT
   <213> Bacillus sp.
<400> 4
<210> 5
   <211> 188
   <212> PRT
   <213> Nocardiopsis sp.
<400> 5
   Ala Asp Ile Ile Gly Gly Leu Ala Tyr Thr Met Gly Gly Arg Cys Ser
   1               5                   10                  15
   Val Gly Phe Ala Ala Thr Asn Ala Ala Gly Gln Pro Gly Phe Val Thr
               20                  25                  30
   Ala Gly His Cys Gly Arg Val Gly Thr Gln Val Thr Ile Gly Asn Gly
           35                  40                  45
   Arg Gly Val Phe Glu Gln Ser Val Phe Pro Gly Asn Asp Ala Ala Phe
       50                  55                  60
   Val Arg Gly Thr Ser Asn Phe Thr Leu Thr Asn Leu Val Ser Arg Tyr
   65                  70                  75                  80
   Asn Thr Gly Gly Tyr Ala Thr Val Ala Gly His Asn Gln Ala Pro Ile
                   85                  90                  95
   Gly Ser Ser Val Cys Arg Ser Gly Ser Thr Thr Gly Trp His Cys Gly
               100                 105                 110
   Thr Ile Gln Ala Arg Gly Gln Ser Val Ser Tyr Pro Glu Gly Thr Val
           115                 120                 125
   Thr Asn Met Thr Arg Thr Thr Val Cys Ala Glu Pro Gly Asp Ser Gly
       130                 135                 140
   Gly Ser Tyr Ile Ser Gly Thr Gln Ala Gln Gly Val Thr Ser Gly Gly
   145                 150                 155                 160
   Ser Gly Asn Cys Arg Thr Gly Gly Thr Thr Phe Tyr Gln Glu Val Thr
   165 170 175
   Pro Met Val Asn Ser Trp Gly Val Arg Leu Arg Thr
   180 185
<210> 6
   <211> 412
   <212> PRT
   <213> Pyrococcus furiosus
<400> 6
   Ala Glu Leu Glu Gly Leu Asp Glu Ser Ala Ala Gln Val Met Ala Thr
   1 5 10 15
   Tyr Val Trp Asn Leu Gly Tyr Asp Gly Ser Gly Ile Thr Ile Gly Ile
   20 25 30
   Ile Asp Thr Gly Ile Asp Ala Ser His Pro Asp Leu Gln Gly Lys Val
   35 40 45
   Ile Gly Trp Val Asp Phe Val Asn Gly Arg Ser Tyr Pro Tyr Asp Asp
   50 55 60
   His Gly His Gly Thr His Val Ala Ser Ile Ala Ala Gly Thr Gly Ala
   65 70 75 80
   Ala Ser Asn Gly Lys Tyr Lys Gly Met Ala Pro Gly Ala Lys Leu Ala
   85 90 95
   Gly Ile Lys Val Leu Gly Ala Asp Gly Ser Gly Ser Ile Ser Thr Ile
   100 105 110
   Ile Lys Gly Val Glu Trp Ala Val Asp Asn Lys Asp Lys Tyr Gly Ile
   115 120 125
   Lys Val Ile Asn Leu Ser Leu Gly Ser Ser Gln Ser Ser Asp Gly Thr
   130 135 140
   Asp Ala Leu Ser Gln Ala Val Asn Ala Ala Trp Asp Ala Gly Leu Val
   145 150 155 160
   Val Val Val Ala Ala Gly Asn Ser Gly Pro Asn Lys Tyr Thr Ile Gly
   165 170 175
   Ser Pro Ala Ala Ala Ser Lys Val Ile Thr Val Gly Ala Val Asp Lys
   180 185 190
   Tyr Asp Val Ile Thr Ser Phe Ser Ser Arg Gly Pro Thr Ala Asp Gly
           195                 200                 205
   Arg Leu Lys Pro Glu Val Val Ala Pro Gly Asn Trp Ile Ile Ala Ala
       210                 215                 220
   Arg Ala Ser Gly Thr Ser Met Gly Gln Pro Ile Asn Asp Tyr Tyr Thr
   225                 230                 235                 240
   Ala Ala Pro Gly Thr Ser Met Ala Thr Pro His Val Ala Gly Ile Ala
                   245                 250                 255
   Ala Leu Leu Leu Gln Ala His Pro Ser Trp Thr Pro Asp Lys Val Lys
               260                 265                 270
   Thr Ala Leu Ile Glu Thr Ala Asp Ile Val Lys Pro Asp Glu Ile Ala
           275                 280                 285
   Asp Ile Ala Tyr Gly Ala Gly Arg Val Asn Ala Tyr Lys Ala Ile Asn
       290                 295                 300
   Tyr Asp Asn Tyr Ala Lys Leu Val Phe Thr Gly Tyr Val Ala Asn Lys
   305                 310                 315                 320
   Gly Ser Gln Thr His Gln Phe Val Ile Ser Gly Ala Ser Phe Val Thr
                   325                 330                 335
   Ala Thr Leu Tyr Trp Asp Asn Ala Asn Ser Asp Leu Asp Leu Tyr Leu
               340                 345                 350
   Tyr Asp Pro Asn Gly Asn Gln Val Asp Tyr Ser Tyr Thr Ala Tyr Tyr
           355                 360                 365
   Gly Phe Glu Lys Val Gly Tyr Tyr Asn Pro Thr Asp Gly Thr Trp Thr
       370                 375                 380
   Ile Lys Val Val Ser Tyr Ser Gly Ser Ala Asn Tyr Gln Val Asp Val
   385                 390                 395                 400
   Val Ser Asp Gly Ser Leu Ser Gln Pro Gly Ser Ser
                   405                 410
<210> 7
   <211> 485
   <212> PRT
   <213> Bacillus sp.
<400> 7
   His His Asn Gly Thr Asn Gly Thr Met Met Gln Tyr Phe Glu Trp Tyr
   1               5                   10                  15
   Leu Pro Asn Asp Gly Asn His Trp Asn Arg Leu Arg Ser Asp Ala Ser
               20                  25                  30
   Asn Leu Lys Asp Lys Gly Ile Ser Ala Val Trp Ile Pro Pro Ala Trp
           35                  40                  45
   Lys Gly Ala Ser Gln Asn Asp Val Gly Tyr Gly Ala Tyr Asp Leu Tyr
       50                  55                  60
   Asp Leu Gly Glu Phe Asn Gln Lys Gly Thr Ile Arg Thr Lys Tyr Gly
   65                  70                  75                  80
   Thr Arg Asn Gln Leu Gln Ala Ala Val Asn Ala Leu Lys Ser Asn Gly
                   85                  90                  95
   Ile Gln Val Tyr Gly Asp Val Val Met Asn His Lys Gly Gly Ala Asp
               100                 105                 110
   Ala Thr Glu Met Val Arg Ala Val Glu Val Asn Pro Asn Asn Arg Asn
           115                 120                 125
   Gln Glu Val Ser Gly Glu Tyr Thr Ile Glu Ala Trp Thr Lys Phe Asp
       130                 135                 140
   Phe Pro Gly Arg Gly Asn Thr His Ser Asn Phe Lys Trp Arg Trp Tyr
   145                 150                 155                 160
   His Phe Asp Gly Val Asp Trp Asp Gln Ser Arg Lys Leu Asn Asn Arg
                   165                 170                 175
   Ile Tyr Lys Phe Arg Gly Asp Gly Lys Gly Trp Asp Trp Glu Val Asp
               180                 185                 190
   Thr Glu Asn Gly Asn Tyr Asp Tyr Leu Met Tyr Ala Asp Ile Asp Met
           195                 200                 205
   Asp His Pro Glu Val Val Asn Glu Leu Arg Asn Trp Gly Val Trp Tyr
       210                 215                 220
   Thr Asn Thr Leu Gly Leu Asp Gly Phe Arg Ile Asp Ala Val Lys His
   225                 230                 235                 240
   Ile Lys Tyr Ser Phe Thr Arg Asp Trp Ile Asn His Val Arg Ser Ala
                   245                 250                 255
   Thr Gly Lys Asn Met Phe Ala Val Ala Glu Phe Trp Lys Asn Asp Leu
               260                 265                 270
   Gly Ala Ile Glu Asn Tyr Leu Asn Lys Thr Asn Trp Asn His Ser Val
           275                 280                 285
   Phe Asp Val Pro Leu His Tyr Asn Leu Tyr Asn Ala Ser Lys Ser Gly
       290                 295                 300
   Gly Asn Tyr Asp Met Arg Gln Ile Phe Asn Gly Thr Val Val Gln Arg
   305                 310                 315                 320
   His Pro Met His Ala Val Thr Phe Val Asp Asn His Asp Ser Gln Pro
                   325                 330                 335
   Glu Glu Ala Leu Glu Ser Phe Val Glu Glu Trp Phe Lys Pro Leu Ala
               340                 345                 350
   Tyr Ala Leu Thr Leu Thr Arg Glu Gln Gly Tyr Pro Ser Val Phe Tyr
           355                 360                 365
   Gly Asp Tyr Tyr Gly Ile Pro Thr His Gly Val Pro Ala Met Lys Ser
       370                 375                 380
   Lys Ile Asp Pro Ile Leu Glu Ala Arg Gln Lys Tyr Ala Tyr Gly Arg
   385                 390                 395                 400
   Gln Asn Asp Tyr Leu Asp His His Asn Ile Ile Gly Trp Thr Arg Glu
                   405                 410                 415
   Gly Asn Thr Ala His Pro Asn Ser Gly Leu Ala Thr Ile Met Ser Asp
               420                 425                 430
   Gly Ala Gly Gly Asn Lys Trp Met Phe Val Gly Arg Asn Lys Ala Gly
           435                 440                 445
   Gln Val Trp Thr Asp Ile Thr Gly Asn Arg Ala Gly Thr Val Thr Ile
       450                 455                 460
   Asn Ala Asp Gly Trp Gly Asn Phe Ser Val Asn Gly Gly Ser Val Ser
   465                 470                 475                 480
   Ile Trp Val Asn Lys
                   485
<210> 8
   <211> 514
   <212> PRT
   <213> Bacillus stearothermophilus
<400> 8
   Ala Pro Phe Asn Gly Thr Met Met Gln Tyr Phe Glu Trp Tyr Leu Pro
   1               5                   10                  15
   Asp Asp Gly Thr Leu Trp Thr Lys Val Ala Asn Glu Ala Asn Asn Leu
               20                  25                  30
   Ser Ser Leu Gly Ile Thr Ala Leu Trp Leu Pro Pro Ala Tyr Lys Gly
           35                  40                  45
   Thr Ser Arg Ser Asp Val Gly Tyr Gly Val Tyr Asp Leu Tyr Asp Leu
       50                  55                  60
   Gly Glu Phe Asn Gln Lys Gly Thr Val Arg Thr Lys Tyr Gly Thr Lys
   65                  70                  75                  80
   Ala Gln Tyr Leu Gln Ala Ile Gln Ala Ala His Ala Ala Gly Met Gln
                   85                  90                  95
   Val Tyr Ala Asp Val Val Phe Asp His Lys Gly Gly Ala Asp Gly Thr
               100                 105                 110
   Glu Trp Val Asp Ala Val Glu Val Asn Pro Ser Asp Arg Asn Gln Glu
           115                 120                 125
   Ile Ser Gly Thr Tyr Gln Ile Gln Ala Trp Thr Lys Phe Asp Phe Pro
       130                 135                 140
   Gly Arg Gly Asn Thr Tyr Ser Ser Phe Lys Trp Arg Trp Tyr His Phe
   145                 150                 155                 160
   Asp Gly Val Asp Trp Asp Glu Ser Arg Lys Leu Ser Arg Ile Tyr Lys
                   165                 170                 175
   Phe Arg Gly Ile Gly Lys Ala Trp Asp Trp Glu Val Asp Thr Glu Asn
               180                 185                 190
   Gly Asn Tyr Asp Tyr Leu Met Tyr Ala Asp Leu Asp Met Asp His Pro
           195                 200                 205
   Glu Val Val Thr Glu Leu Lys Asn Trp Gly Lys Trp Tyr Val Asn Thr
       210                 215                 220
   Thr Asn Ile Asp Gly Phe Arg Leu Asp Ala Val Lys His Ile Lys Phe
   225 230 235 240
   Ser Phe Phe Pro Asp Trp Leu Ser Tyr Val Arg Ser Gln Thr Gly Lys
   245 250 255
   Pro Leu Phe Thr Val Gly Glu Tyr Trp Ser Tyr Asp Ile Asn Lys Leu
   260 265 270
   His Asn Tyr Ile Thr Lys Thr Asn Gly Thr Met Ser Leu Phe Asp Ala
   275 280 285
   Pro Leu His Asn Lys Phe Tyr Thr Ala Ser Lys Ser Gly Gly Ala Phe
   290 295 300
   Asp Met Arg Thr Leu Met Thr Asn Thr Leu Met Lys Asp Gln Pro Thr
   305 310 315 320
   Leu Ala Val Thr Phe Val Asp Asn His Asp Thr Glu Pro Gly Gln Ala
   325 330 335
   Leu Gln Ser Trp Val Asp Pro Trp Phe Lys Pro Leu Ala Tyr Ala Phe
   340 345 350
   Ile Leu Thr Arg Gln Glu Gly Tyr Pro Cys Val Phe Tyr Gly Asp Tyr
   355 360 365
   Tyr Gly Ile Pro Gln Tyr Asn Ile Pro Ser Leu Lys Ser Lys Ile Asp
   370 375 380
   Pro Leu Leu Ile Ala Arg Arg Asp Tyr Ala Tyr Gly Thr Gln His Asp
   385 390 395 400
   Tyr Leu Asp His Ser Asp Ile Ile Gly Trp Thr Arg Glu Gly Val Thr
   405 410 415
   Glu Lys Pro Gly Ser Gly Leu Ala Ala Leu Ile Thr Asp Gly Pro Gly
   420 425 430
   Gly Ser Lys Trp Met Tyr Val Gly Lys Gln His Ala Gly Lys Val Phe
   435 440 445
   Tyr Asp Leu Thr Gly Asn Arg Ser Asp Thr Val Thr Ile Asn Ser Asp
   450 455 460
   Gly Trp Gly Glu Phe Lys Val Asn Gly Gly Ser Val Ser Val Trp Val
   465 470 475 480
   Pro Arg Lys Thr Thr Val Ser Thr Ile Ala Arg Pro Ile Thr Thr Arg
   485 490 495
   Pro Trp Thr Gly Glu Phe Val Arg Trp Thr Glu Pro Arg Leu Val Ala
   500 505 510
   Trp Pro
<210> 9
   <211> 483
   <212> PRT
   <213> Bacillus licheniformis
<400> 9